# EUROPEAN PATENT SPECIFICATION

(11) **EP 0 623 399 B1**
(45) Date of publication and mention of the grant of the patent: **12.08.1998**
(21) Application number: 94106837.1
(22) Date of filing: 02.05.1994
(51) Int. Cl.: B09B 1/00, B09B 3/00

(54) **Process for the disposal of waste, particularly municipal solid waste**
Verfahren zur Entsorgung von Abfällen, insbesondere von Hausmüll
Procédé pour l'élimination de déchets, en particulier pour des déchets urbains solides

(30) Priority: 05.05.1993 IT MI930890
(43) Date of publication of application: 09.11.1994
(73) Proprietor: ECODECO S.p.A., I-27010 Giussago (Pavia) (IT)
(72) Inventor: Calcaterra, Enrico, I-20073 Codogno (Milano) (IT); Natta, Giuseppe, I-27010 Giussago (Pavia) (IT); Tugnoli, Marco, I-27014 Corteolona (Pavia) (IT)
(74) Representative: Forattini, Amelia

(56) References cited:
- EP-A- 0 373 460
- DE-A- 1 940 434
- DE-A- 4 018 810

## Description

The present invention relates to a process for the disposal of waste.

As is known, the controlled dumping site is the system used most frequently, at least in Italy, for the disposal of municipal solid waste, because of its low cost and also because of the difficulties encountered in providing new incinerators or in reconditioning and adapting existing ones.

However, this solution entails a series of well-known drawbacks for the environment and for the quality of life of the population that inhabits the area in the vicinity of the dumping site.

The waste placed in dumping sites will in fact ferment and putrefy. The percentage of organic material (pure organic material plus paper and cardboard) can reach 60% of the untreated waste and is in any case not less than 30% even in waste from areas where differentiated waste collection has been practiced for several years. The main drawbacks that characterize the disposal of waste in a dumping site are very briefly listed hereafter:
-- The large areas required to accommodate waste. Due to the limited density obtained with conventional compacting systems, there is an intense "consumption" of suitable sites, which are increasingly difficult to find.

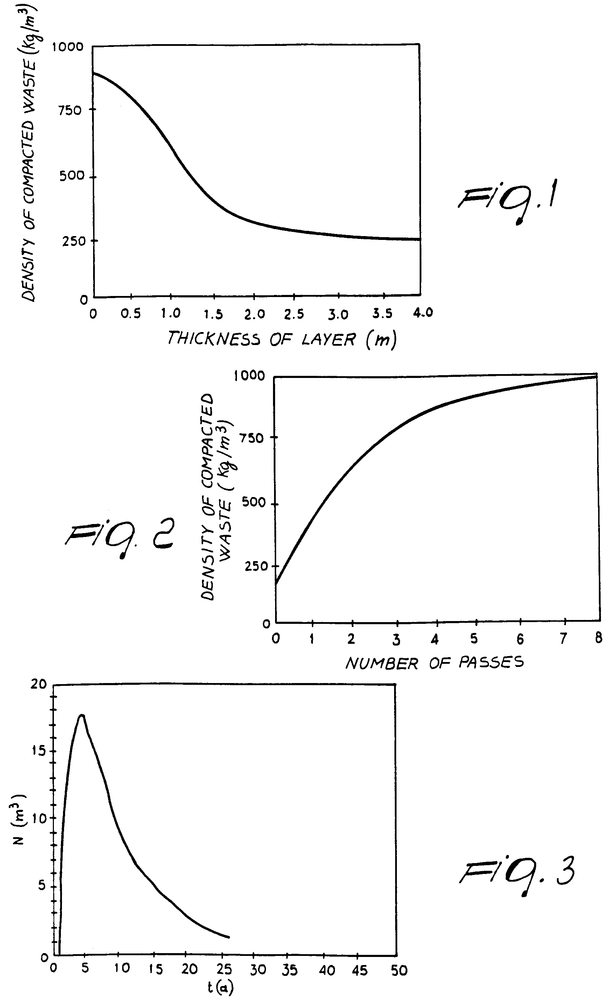

   Figures 1 and 2 illustrate, by way of example, the variations in the density of the layers of waste as a function of height and of the number of passes made with a compacting device.
-- The release of unpleasant odors and the dispersion of waste material even in neighboring areas with the consequent spoiling of the landscape. The problem of odorous emissions is the greatest deterrent to the possibility of creating dumping sites.
   Daily covering of the refuse and other systems, even with the best management of the site, have proved inadequate to curb the problem, which can become a nuisance over a wide area especially in the warmer periods of the year.
   The "light" materials carried by the wind are also a cause of problems, and the costs for their recovery may be significant.
-- The spread of vermin and pests. In addition to directly affecting civil and industrial activities, rats, sea-gulls, flies etc. act as vectors for human and animal infections.
-- The production of biogas. An aspect that is often neglected in evaluating the environmental impact of dumping sites is the diffusion of biogas both in the ground and in the surrounding air. This occurs because of the low intrinsic efficiency of the capture systems, which even in the best of cases is never higher than 50-60%.
   Biogas is normally produced starting from the second year of activity of the dumping site and continues for 30-35 years. A typical trend of biogas production in the course of time is shown in Figure 3.
   The captured biogas is only rarely recovered as an energy source; in most cases it is burned in a torch.
   Mention should also be made of the drawbacks that biogas can produce in vegetation, due both to the anoxia caused by oxygen replacement and to the presence of plant-poisoning compounds in the biogas. These conditions ultimately can delay or even prevent the eventual environmental reclamation of the dumping site.
-- The production of percolate. This production depends on several factors, such as rainfall, surface water flow, evaporation/transpiration, and the variation in humidity of the waste placed in the site and of the intermediate covering layers. Percolate production normally varies between 3 and 10 m³/ha/day as a function of rainfall, dumping site age and degree of compaction. The disposal cost of this percolate can be very high.

EP-A-373460 discloses a waste disposal system which provides for compressing the waste and wrapping it in plastic foil for orderly stacking.

DE-A-4018810 discloses a process for composting household rubbish containing organic material comprising a composting step followed by a compression using clamp in closed containers.

DE-A-1940434 discloses a process for waste disposal from households and small business, including a grinding rubbish on site, drying at 120°C, pressing and waterproof packing.

The aim of the present invention is to provide a system that is suitable to solve the above described problems.

Within the scope of this aim, an object of the invention is to provide a process for recycling the waste as fuel.

A further object of the invention is to provide an economical process.

This aim, this object and others which will become apparent hereinafter are achieved by a process for the disposal of waste, as claimed in the independent appended claims.

Further characteristics and advantages will become apparent from the description of a preferred but not exclusive embodiment of the invention, illustrated only by way of non-limitative example in the accompanying drawings, wherein:
Figure 1 plots the density of the waste in the dumping site as a function of layer height;
Figure 2 plots the density of the waste in the dumping site as a function of the number of passes in the compaction device;
Figure 3 plots the typical trend of biogas production in the dumping site as a function of time;
Figure 4 is a schematic view of the steps of the process according to the invention;
Figure 5 is a schematic view of the step of depositing the waste in the dumping site;

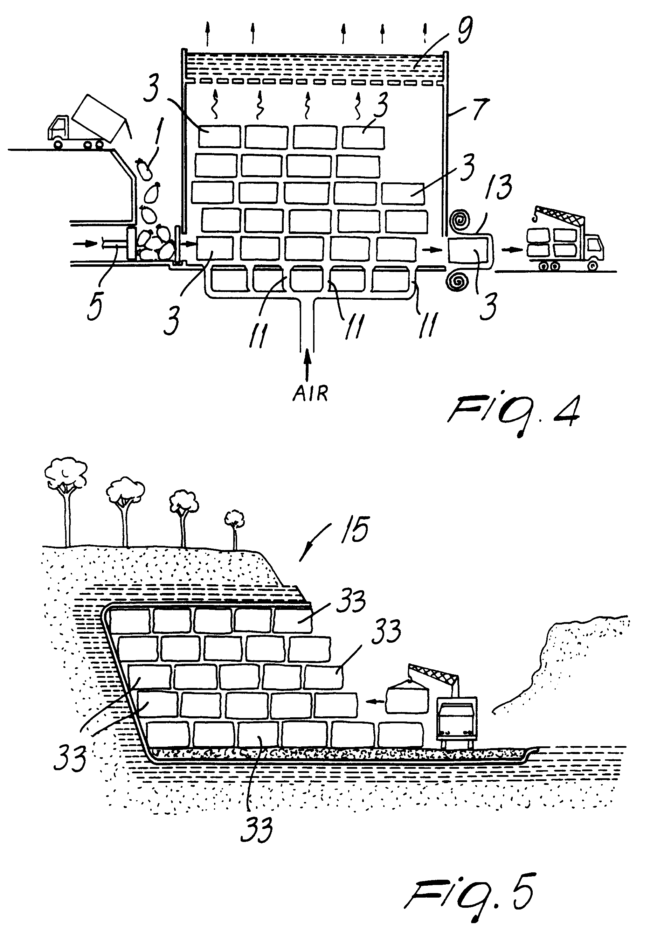

With reference to Figures 4 and 5, the process according to the invention first includes homogenising and grinding the refuse.

Ferrous components may be extracted from the refuse at this stage, if required.

According to a first aspect of the invention the graded refuse is then formed into bales having a high resistance to pressure.

For this purpose it is possible to use presses 5 which are suitable to compact solid waste 1 and may operate continuously or discontinuously, producing bales 3 that have a prismatic shape of constant height and depth and variable length. In the press, the pressure is constant and is applied along the horizontal axis. In this manner, average pressures of 10 to 30 kg/cm² are achieved inside the bales, and a bale has the maximum ability to withstand the pressure of many overlying bales, and accordingly to avoid deformation, along the direction in which pressure is applied.

The process according to the invention also includes a step of treating the waste in the bales by aerobic fermentation. This treatment may be performed either "naturally" or "rapidly". The "natural" treatment is performed directly in the bales which contain grated material. The "rapid", or accelerated, treatment is performed in reactors (tanks) suitably prepared before the bales are formed, as illustrated hereafter.

The "natural" fermentation treatment comprises placing the bales 3 inside a closed building, for example a large shed 7, provided with a biofilter 9.

The bales 3 can advantageously be arranged in multiple superimposed layers, by means of an adapted automatic device for example, according to a specific layout in which a distance of 9-10 cm is kept between the bales to facilitate the escape of air and allow oxygenation of the outer layers of the bales, which is required for their aerobic fermentation.

Air is changed through a flooring system that is appropriately provided with ventilation ports 11 connected to external air intakes, using the "chimney effect" of the air heated by the warm bales. For the initial step, and when these conditions do not occur, the shed 7 can also be provided with a suitable system for artificial injection and aspiration.

The outer layer of the bales, due to aerobic microbial activity, reaches high temperatures (50-60°C) within a few days. This outer layer (approximately 20-25 cm) can constitute as much as 70% of the total volume of the bale, depending on its dimensions and shape. The remaining internal part undergoes anaerobic fermentation processes; the unpleasant odors that are typical of these reactions are mostly degraded by the activity of the aerobic outer layers (biofilter effect). However, in order to eliminate any possibility of emission of odors from the facility, a system of biofilters 9 arranged above the covering of the shed treats the air.

The outer layer of the bales, by reaching high temperatures, consumes a lot of water; water is accordingly drawn toward the outside from the inner core, accelerating the drying of the core. In this manner, after 120-150 days the bales dehydrate until they reach a level that is incompatible with microbiological activities. At the same time, the temperature also drops until it settles at a value close to ambient temperature. During this step, the bale has lost approximately 15-20% of its original weight.

According to a second aspect of the invention, after the refuse has been homogenized and graded and before it is formed into bales, the refuse undergoes a "rapid" fermentation treatment. To this purpose, the refuse is advantageously arranged inside tanks and subjected to aerobic fermentation by deodorizing and dehydration. The dehydrated refuse may also be subjected to any conventional chemical, physical or microbiological treatment for eliminating any residual microbiological (methanogen) activity.
The refuse is then formed into bales as described above.

According to both aspects of the invention, if the bales are impermeabilized, as illustrated hereinafter, any additional fermentative microbial activity is prevented inside it. If the threshold at which the fermentative microbial (methanogen) activity is blocked is not reached, simply-fashioned valves are added during impermeabilization to allow the venting of any residual gas micro-productions.

Finally, the process, according to both aspects of the invention, includes a step for impermeabilizing the bales. This step entails the forming of a system for impermeabilizing waste compacted in uniform bales that still have a high content of organic substances but are in such a state of desiccation as to prevent the continuation or the resumption of fermentative microbial activities.

Impermeabilization is advantageously performed by using films 13 made of plastic material or of other suitable materials that in any case have the characteristic of not allowing rehydration of the waste.

The last step of the process includes the placement of the impermeabilized bales 33 in a dumping site, as shown in Figure 5.

Other advantages obtained with the impermeable wrapping are the elimination of the problem of percolate and of the dispersion of light waste materials into the surrounding environment.

Among the materials suitable to perform impermeabilization, mention is made by way of example of plastic films, either of the shrink-wrap type or of the conventional type that are fully hermetic or have openings that act as vent valves, and bituminous films.

In practice it has been observed that the invention achieves the intended aim and objects, providing a disposal system that can solve the problems of conventional systems.

The process according to the invention is susceptible to numerous modifications and variations, all of which are within the scope of the inventive concept;all the details may furthermore be replaced with technically equivalent items.

The materials employed, as well as the dimensions, may naturally be any according to the requirements and the state of the art.

## Claims

1. A process for the disposal of waste, comprising the steps of:
- grinding said waste;
- forming said waste into bales (3) having a high resistance to pressure;
characterized in that it further comprises treating said waste by aerobic fermentation for a period of time sufficient to allow said bales to dehydrate until they reach a level that is incompatible with microbiological activities, by:
- placing said bales in a closed building (7) in multiple stacked layers;
- injecting air in said building; said multiple stacked layers being adapted to allow the escape of said injected air and the oxygenation of the outer layers of the bales; said bales being finally removed from said closed building and placed in a dumping site (15).

2. Process according to claim 1, characterized in that said bales are arranged at a distance of approximately 8 to 10 cm from each other.

3. Process according to one or more of the preceding claims, characterized in that said closed building comprises a biofilter (9) which is arranged above the covering of said building to treat the air.

4. Process according to one or more of the preceding claims, characterized in that it comprises the impermeabilization of said bales before said bales are placed in said dumping site.

5. A device as used in the performance of the method for treating waste of any of the preceding claims, comprising grinding and baling equipment and a closed fermentation space, characterized in that said space is a building (7) suitable to accommodate said waste, and comprising a roof with a biofilter (9), and a flooring system provided with air passages (11).

6. The device according to claim 5, characterized in that it comprises a press that is suitable to press said waste to produce said bales.

7. The device according to claim 5 or 6, characterized in that it comprises an impermeabilization means that is suitable to wrap said bales in an impermeable film (13).

## Patentansprüche

1. Verfahren zur Entsorgung von Abfällen, insbesondere von Hausmüll, mit den Schritten
- Zerkleinern der Abfälle und
- Formen der Abfälle in Ballen (3) mit hohem Druckwiderstand,
**dadurch gekennzeichnet,**
daß es weiterhin umfaßt die Behandlung der Abfälle durch aerobe Fermentation für eine Zeitdauer, die ausreichend ist, um die Dehydrierung der Ballen zuzulassen, bis zu einem Level der imkompatibel mit mikrobiologischen Aktivitäten ist, in dem
- die Ballen in einem geschlossenen Gebäude (7) in einer Vielzahl von Lagerschichten positioniert werden,
- Luft in das Gebäude eingeblasen wird, wobei die vielschichtig gelagerten Lagen das Entweichen der eingeblasenen Luft und die Oxydierung der äußeren Schichten der Ballen ermöglichen, und
- die Ballen schließlich aus dem geschlossenen Gebäude entfernt und auf einem Ablagerungsplatz (15) plaziert werden.

2. Verfahren nach Anspruch 1, dadurch gekennzeichnet, daß die Ballen mit einem Abstand von etwa 8 bis 10 cm voneinander angeordnet werden.

3. Verfahren nach einem der vorhergehenden Ansprüche, dadurch gekennzeichnet, daß das geschlossene Gebäude einen Biofilter (9) aufweist, welcher auf der Abdeckung des Gebäudes zur Behandlung der Luft angeordnet ist.

4. Verfahren nach einem der vorhergehenden Ansprüche, dadurch gekennzeichnet, daß die Ballen vor der Plazierung am Ablagerungsplatz abgedichtet werden.

5. Vorrichtung zur Durchführung des Verfahrens zur Entsorgung von Abfällen nach einem der vorhergehenden Ansprüche, umfassend Zerkleinerungs- und Paketierungsausrüstung und einen geschlossenen Fermentierungsbereich, dadurch gekennzeichnet, daß der Bereich ein Gebäude (7) ist, geeignet zur Aufnahme der Abfälle, mit einem Dach mit einem Biofilter (9) und einem Bodensystem mit Luftdurchlässen (11).

6. Vorrichtung nach Anspruch 5, dadurch gekennzeichnet, daß diese eine Presse aufweist, geeignet zur Pressung der Abfälle zur Herstellung der Ballen.

7. Vorrichtung nach einem der Ansprüche 5 oder 6, dadurch gekennzeichnet, daß diese eine Abdichtungsvorrichtung aufweist, geeignet zum Packen der Ballen in einen undurchlässigen Film (13).

## Revendications

1. Procédé de rejet des déchets, comportant les étapes consistant à :
- meuler lesdits déchets ;
- former lesdits déchets en ballots (3) ayant une résistance élevée à la pression ;
caractérisé en ce qu'il comporte de plus l'étape consistant à traiter lesdits déchets par fermentation aérobie pendant une période de temps suffisante pour permettre auxdits ballots de se déshydrater jusqu'à ce qu'ils atteignent un niveau qui est incompatible avec les activités microbiologiques, en :
- plaçant lesdits ballots dans une construction fermée (7) à multiples couches empilées ;
- injectant de l'air dans ladite construction ; lesdites multiples couches empilées étant adaptées pour permettre l'échappement dudit air injecté et l'oxygénation des couches extérieures des ballots ; lesdits ballots étant finalement enlevés de ladite construction fermée et placés dans un site de décharge (15).

2. Procédé selon la revendication 1, caractérisé en ce que lesdits ballots sont agencés à une distance d'approximativement 8 à 10 cm les uns des autres.

3. Procédé selon l'une quelconque des revendications précédentes, caractérisé en ce que ladite construction fermée comporte un filtre biologique (9) qui est agencé au-dessus du recouvrement de ladite construction pour traiter l'air.

4. Procédé selon l'une quelconque des revendications précédentes, caractérisé en ce qu'il comporte l'imperméabilisation desdits ballots avant que les ballots ne soient placés dans ledit site de décharge.

5. Dispositif tel qu'utilisé dans la mise en oeuvre du procédé pour traiter les déchets selon l'une quelconque des revendications précédentes, comportant un équipement de meulage et de mise en ballots et un espace de fermentation fermé, caractérisé en ce que ledit espace est une construction (7) adaptée pour recevoir lesdits déchets, et comportant un toit muni d'un filtre biologique (9), et d'un système de plancher muni de passages d'air (11).

6. Dispositif selon la revendication 5, caractérisé en ce qu'il comporte une presse qui est adaptée pour presser lesdits déchets pour produire lesdits ballots.

7. Dispositif selon la revendication 5 ou 6, caractérisé en ce qu'il comporte des moyens d'imperméabilisation qui sont adaptés pour enrouler lesdits ballots dans un film imperméable (13).
